(19)

Europäisches Patentamt
European Patent Office
Office européen des brevets

(11) **EP 4 371 477 A1**

(12) **EUROPEAN PATENT APPLICATION**

(43) Date of publication:
**22.05.2024 Bulletin 2024/21**

(21) Application number: **23210769.8**

(22) Date of filing: **17.11.2023**

(51) International Patent Classification (IPC):
**A61B 5/103** (2006.01)        **A61B 5/00** (2006.01)

(52) Cooperative Patent Classification (CPC):
**A61B 5/1034; A61B 5/14517; A61B 5/4266;
A61B 5/443; A61B 5/6829; G01J 3/52;
G01N 21/78; G06T 7/90;** G01N 2021/1765

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.11.2022 GB 202217251**

(71) Applicant: **Skyrocket Phytopharma Ltd.
Harlow Essex CM17 9TX (GB)**

(72) Inventor: **Simpson, John
Essex, CM17 9TX (GB)**

(74) Representative: **Barker Brettell LLP
100 Hagley Road
Edgbaston
Birmingham, West Midlands B16 8QQ (GB)**

(54) **SYSTEM FOR DETERMINING SKIN DRYNESS**

(57)     A system for use in determining a skin dryness by processing images of a test patch of the kind comprising a membrane impregnated with a moisture indicator which has a first colour in the dry anhydrous state, and which changes to a second colour at a certain moisture content which corresponds to normal skin moisture comprises a user operable computer device having a camera, a processor, and an area of memory, a computer program stored in the memory of the user operable device, and a scorecard having at least one colour reference target. The computer program causes the user operable computer device to process an image of the scorecard with a test patch placed on it after the test patch has been applied to an area of skin, the processing determining the dryness of the area of the skin.

Figure 1

**Description**

The invention relates to a system for determining skin dryness in a consistent and reliable manner, and to a method of determining an abnormal skin dryness.

There are a number of health conditions that can lead to abnormally dry skin. A common but serious condition is Diabetes and it is well known that related foot ulcers and amputations are serious complications of poorly controlled diabetes. It is estimated that many people with diabetes are not even aware that foot problems are a common complication. Because of this the warning signs that problems are forming are often missed.

Damage to the nerves in the feet because of diabetes can result in the sweat glands not producing enough moisture, leading to dry and cracked feet. The medical term for this is sudomotor dysfunction.

In European patent No EP127499B there is taught a method for determining abnormal skin dryness, in which a particularly textile reaction carrier is impregnated with a 3.15 to 25 percent cobalt(II) chloride solution and then dried until the cobalt(II) chloride turns blue , the reaction carrier is attached to one side of a flat base body made of a flexible material, the reaction carrier is covered with an airtight and moisture-impermeable protective material, the protective material immediately in front the determination of the skin dryness is removed and the reaction carrier is placed on a skin area and fixed and after a predetermined period of time is removed from the skin.

The textile carrier, in the form of an adhesive patch is used to determine the liquid content of the sweat on a skin area, for example the sole of the foot. To perform a test, it is known for the user to stick a patch to the sole of each foot like a sticking plaster and left in place for 10 minutes. The patch is blue to start with and should turn pink, in the presence of moisture from sweating, to indicate a normal result.

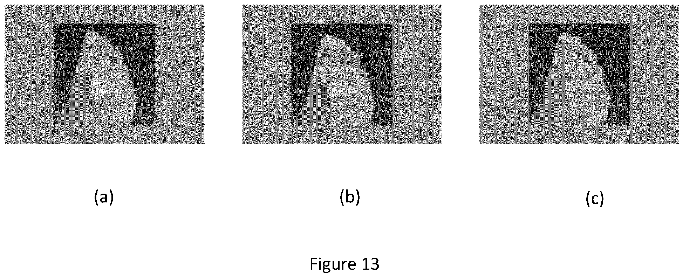

A test result for a normal foot is shown in Figure 13(a) and is pink. If the test patch stays blue as shown in Figure 13(c) , or if it turns a patchy blue/pink as shown in Figure 13(b), then this indicates that the person may have some level of nerve damage and that their sweat glands are not working properly as there is not enough moisture to complete the colour change.

Whilst these patches are simple to apply and to use for the extreme cases of a normal foot and for one with high levels of sudomotor dysfunction, it is not easy for a user to track mild symptoms as the pink-blue colour change is subjective.

According to a first aspect the invention provides a system for use in determining an 1 skin dryness by processing images of a test patch of the kind comprising a membrane impregnated with a moisture indicator which has a first colour in the dry anhydrous state, and which changes to a second colour at a certain moisture content which corresponds to normal skin moisture, the system comprising:

A user operable computer device having a camera, a processor, and an area of memory, a computer program stored in the memory of the user operable device,
a scorecard having at least one colour reference target;
in which the computer program causes the user operable computer device to process an image of the scorecard with a test patch placed on it after the test patch has been applied to an area of skin, the processing determining a dryness of the area of the skin.

The system may in particular determine whether an area of skin is abnormally dry, for example the skin on a foot. The dryness of a foot can be used as an indicator of the presence and severity of a range of conditions including diabetes.

The moisture indicator may comprise cobalt (II) chloride which has blue colour when in the dry anhydrous state and a pink colour when at the certain moisture content. The colour reference target may comprise first target which has a colour that corresponds to a fully dehydrated test patch and a second target having a colour that corresponds to a fully hydrated test patch and a region in which a test patch can be placed.

The system may be arranged to process the captured image to extract a two-dimensional image of the test patch which is colour balanced using the colours of the colour reference target or targets as seen in the captured image. Because these targets are known to be the colour of a fully dehydrated and a fully hydrated pixel in the image of the patch the processing means may determine an overall percentage colour for the patch by comparing the colour of each pixel with the colour of both targets.

The results may comprise a percentage value that is calculated as:

$$\text{Percentage} = \frac{\text{pixels deemed blue}}{\text{pixels deemed blue} + \text{pixels deemed pink}} * 100$$

Additionally or alternatively the colour reference target may include a colour swatch including a region of cyan colour, a region of magenta colour, and a region of yellow colour.

In this case the system may process the regions of the image corresponding to the colour swatch so as to colour balance the image of the scorecard.

The scorecard may further include one or more fiducial markers that have a known shape or pattern, optionally a known size and optionally a known location on the scorecard and the processing may use the fiducial markers to produce a modified image of the scorecard which is corrected for perspective and brought into focus. In effect the image is modified until the shape of the markers in the modified image match the known shape of the marker. In an example where the marker is known to be square, an image captured with perspective will have the marker appear to be not square and the modification will ensure the marker is square in the modified image. This modification of the image to correct for distortion effects of perspective- or even correct distortion due to the lens of the image capturing device- may be performed by the system before the location and colour of the moisture indicator are determined, and before or after colour correction of the image using the colour reference target.

Removing the effect of perspective and sharpening the focus of the image may improve the accuracy of the subsequent location of the region of moisture indicator and determining the colour of the moisture indicator.

The system may be configured to store in a database in the memory captured images captured over a period of time using the camera, and to store alongside each image the results of the processing of the captured image.

Alternatively, the computer program may cause the results but not the captured images to be stored in the database.

The computer program may cause the processor to process the results in the database to determine if the abnormality is changing over time. In the even that a change is detected over time a message or alarm may be presented to the user. This may be displayed on a screen of the computer device for example.

The system may be arranged to transmit the captured images, and optionally the processed data, across a network connection to a remote server. This may be used to send the data to a physician who may interpret the captured images or the results.

The computer program may comprise an app, of the kind that can be downloaded from an app store such as those provided by Google (Registered trademark) or Apple (Registered Trademark).

The user operable device may comprise a smartphone.

The scorecard plays a key role in the processing of the images, where images may be captured in very different lighting condition from one image to the next.

The scorecard may comprise a backing portion having a front face which is provided with markings defining an area of the front face to which a test patch can be adhered, and the first and second reference targets.

In addition, the front face may be provided with at least one grey calibration scale.

The backing portion may be white in the vicinity of the marking and area where the patch is fixed.

The front face may be provided with an adhesive portion that is indicated by the markings and which in use sticks to the rear side of a test patch to hold the test patch in position on the scorecard.

The first and second targets may have any known shape, but should not be of the same shape and size as the patch so that they are not mixed up when the image is captured. For example, the patch may be generally rectangular or square and the reference targets may each be triangular.

The system may process the captured images of the scorecard and the test patch to determine the location of the test patch by identifying the location and orientation of the targets.

The test patches may comprise a substrate comprising transparent polyolefin film and a hypoallergenic adhesive. The membrane may be permeable and impregnated with the moisture indicator in the form of a solution, for example a $CoCl_2$ solution.

A suitable patch has been made available to the markets under the brand name Neuropad (Registered Trademark) for example. This patch may be form part of the system of the present invention.

The system may be arranged to process the captured image to extract a two-dimensional image of the test patch which is colour balanced using the colours of the two targets as seen in the captured image. Because these targets are known to be the colour of a fully dehydrated and a fully hydrated pixel in the image of the patch the processing means may determine an overall percentage colour for the patch by comparing the colour of each pixel with the colour of both targets.

Whilst the invention of the first aspect processes the image on the user device it is within the scope of the invention that the processing is performed remotely.

Accordingly, within a further aspect the invention provides a system for use in determining a skin dryness by processing images of a test patch of the kind comprising a membrane impregnated with a moisture indicator, which has a first colour in the dry anhydrous state, and which changes to a second colour at a certain moisture content which corresponds to normal skin moisture, the system comprising:

A user operable computer device having a camera, a processor and an area of memory, a computer program stored in the memory of the user operable device,

a scorecard having at least one colour reference target, for example a first target which has a colour that corresponds to a fully dehydrated test patch and a second target having a colour that corresponds to a fully hydrated test patch and a region in which a test patch can be placed, and ;

in which the computer program causes the user operable computer device to capture an image of the scorecard with a test patch placed on it after the test patch has been applied to an area of skin such as the foot of a patient and to transmit the captured image across a network to a remote server that is configured to process the image to determine whether a level of dryness of the skin..

According to a third aspect the invention provides a method of determining an skin dryness by processing images of a test patch of the kind comprising a membrane impregnated with a moisture indicator, which has a first colour in the dry anhydrous state, and which changes to a second colour at a certain moisture content which corresponds to normal skin moisture, the method comprising:

capturing an image of a test patch that has previously been stuck to an area of skin together with an image of a scorecard, the scorecard having at least one colour reference target and a region in which a test patch can be placed;

processing the captured image to identify the colour reference target and the patch;

calibrating the colours in the captured image using the colours of the captured target, and

analysing the image of the patch in the captured image to determine the percentage of the patch that has the first colour and the second colour.

The method may comprise capturing the image on a portable device such as a smart phone.

The steps of processing the captured image, and calibrating and analysing may each be performed using the processor of the mobile device.

In an alternative the method may comprise transmitting the captured image to a remote server over a network, causing the server to perform the steps and subsequently transmitting a response to the local user device.

The method may comprise fixing the test patch to a scorecard prior to capturing the image at a location on the scorecard indicated by indicia or markings on the scorecard.

The method may comprise prior to the steps listed above attaching a test patch to a foot for a known duration of time before removing the patch for analysis.

The colour reference target may comprise first target which has a colour that corresponds to a fully dehydrated test patch and a second target having a colour that corresponds to a fully hydrated test patch and a region in which a test patch can be placed and the method may comprise processing the captured image to extract a two-dimensional image of the test patch which is colour balanced using the colours of the colour reference target or targets as seen in the captured image.

Additionally or alternatively the colour reference target may include a colour swatch including a region of cyan colour, a region of magenta colour, and a region of yellow colour and the method may comprise processing the regions of the image corresponding to the colour swatch so as to colour balance the image of the scorecard.

The scorecard may further include one or more fiducial markers that have a known shape or pattern on the scorecard and the processing may use the fiducial markers to produce a modified image of the scorecard which is corrected for perspective and brought into focus. This may be performed by the system before the location and colour of the moisture indicator are determined, and before or after colour correction of the image using the colour reference target.

According to a fourth aspect the invention provides a computer program which when operable on a user device, such as a mobile phone, causes the device to perform the method of the third aspect of the invention.

According to a fifth aspect the invention provides a scorecard for use in combination with a test patch of the kind comprising a membrane impregnated with a moisture indicator, which has a first colour in the dry anhydrous state, and which changes to a second colour at a certain moisture content which corresponds to normal skin moisture, , the scorecard comprising:

a backing portion;
at least one colour reference target, and
and in which the backing portion includes a region that is sized to receive a test patch.

The colour reference target may comprise a first reference target having a known shape and having a colour corresponding to a colour of the test patch when fully dehydrated; a second reference target having a known shape and having a colour corresponding to a colour of the test patch when fully dehydrated,

Additionally or alternatively the colour reference target may include a colour swatch including a region of cyan colour, a region of magenta colour, and a region of yellow colour and the method may comprise processing the regions of the image corresponding to the colour swatch so as to colour balance the image of the scorecard.

The scorecard may further include one or more fiducial markers that have a known shape or pattern on the scorecard and the processing may use the fiducial markers to produce a modified image of the scorecard which is corrected for perspective and brought into focus. This may be performed by the system before the location and colour of the moisture indicator are determined, and before or after colour correction of the image using the colour reference target.

The scorecard may include an adhesive portion that is spaced from the two reference targets that is configured to receive and adhere to a patch.

The region for receiving the test patch may be identified by markings on the scorecard.

The targets may have any known shape, but should not be of the same shape and size as the patch so that they are not mixed up when the image is captured. For example, the patch may be generally rectangular or square and the reference targets may be triangular.

One target may match the first colour of the moisture indicator, for example blue and the other target may match the second colour of the moisture indicator, for example pink.

According to a sixth aspect the invention provides a test kit comprising at least one scorecard and at least one compatible test patch.

The test kit may include instructions on where to find a computer App for use with the scorecard. This may be in the form of a world wide web address (URL) that the user can type into a web browser, or a QR code that when read by a suitable QR reader directs a user to a location from which they can download the App to a mobile device such as a smartphone.

The instructions may be printed onto the scorecard, or may be printed onto packaging or onto an instruction leaflet provided with the kit.

The skilled person will appreciate that the system and method and other aspects of the invention may be used to determine an abnormal dryness of an area of skin of a person. This may provide an indication of the presence or severity of a range of conditions including but not limited to: Fabry disease, Guillain-Barre Syndrome, Parkinson's disease, Alzheimer's disease, Chemotherapy induced peripheral neuropathy, Human Immunodeficiency Virus infection, Cardiovascular Autonomic Neuropathy, Sjogren's disease and Lupus.

The invention may also be used with synthetic skins or human skin as a test for the efficiency of anti-perspirants as a means for reducing or preventing perspiration of a skin without any modification of the system or method or computer program or scorecard. In this case, an antiperspirant may be applied to skin under a set of controlled conditions and the change in moisture content of a path or patches observed over time.

There will now be described, by way of example only, one embodiment of the present invention with reference to and as illustrated in the accompanying drawings of which:

**Figure 1** is an overview of a system according to a first aspect of the invention;

**Figure 2** is a view of an alternative system to that of Figure 1;

**Figure 3** is view of a scorecard that can be used in the system of Figure 1 or Figure 2;

**Figure 4** is a plan view of a test patch for use in the system of Figure 1 or Figure 2;

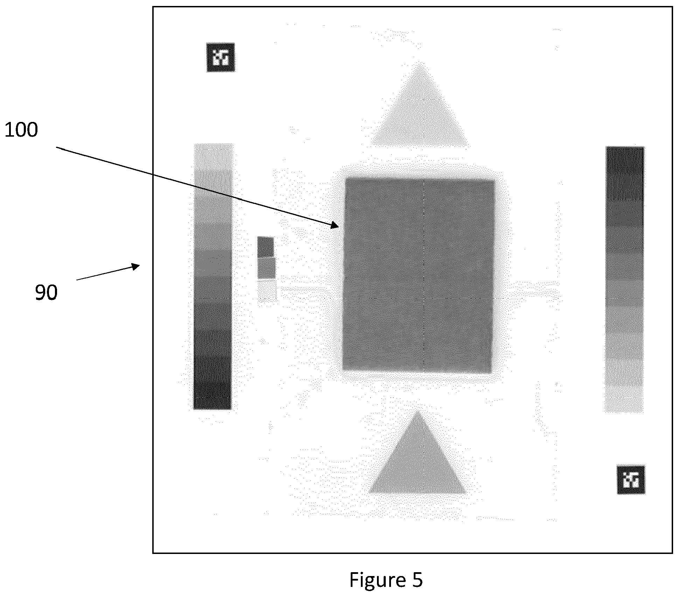

**Figure 5** is a captured image of the test patch of Figure 4 applied to the scorecard of Figure 3;

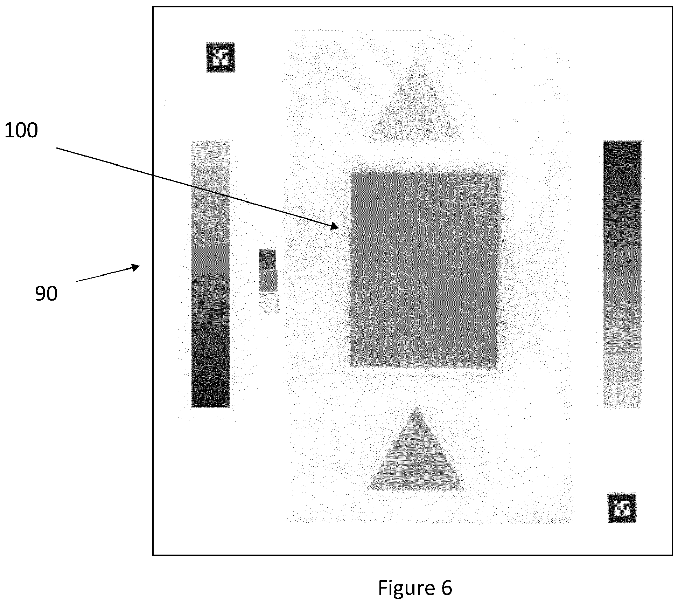

**Figure 6** is a processed image generated from the image of Figure 5 in which the location of the targets and test patch have been enhanced and the image resized and in which the test patch is blue indicating an abnormal level of hydrosis;

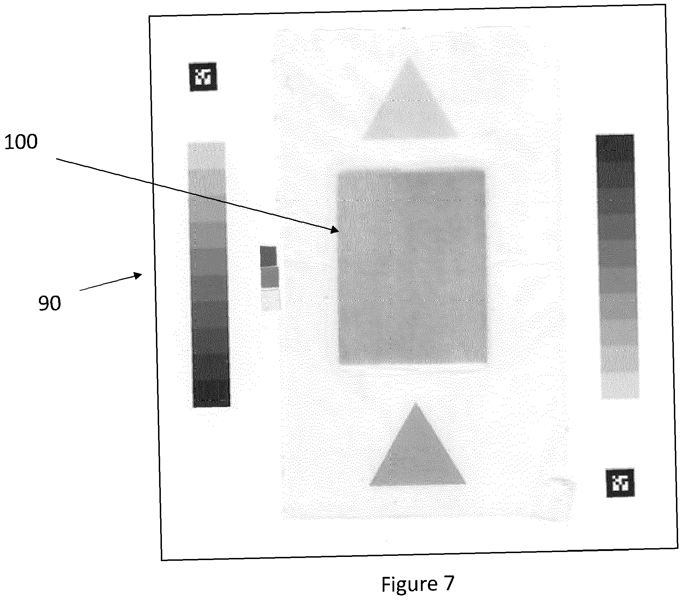

**Figure 7** is a processed image corresponding to Figure 6 in which the test patch has turned pink-blue after applying to a foot for the predefined time indicating some abnormality but milder than for that of Figure 6;

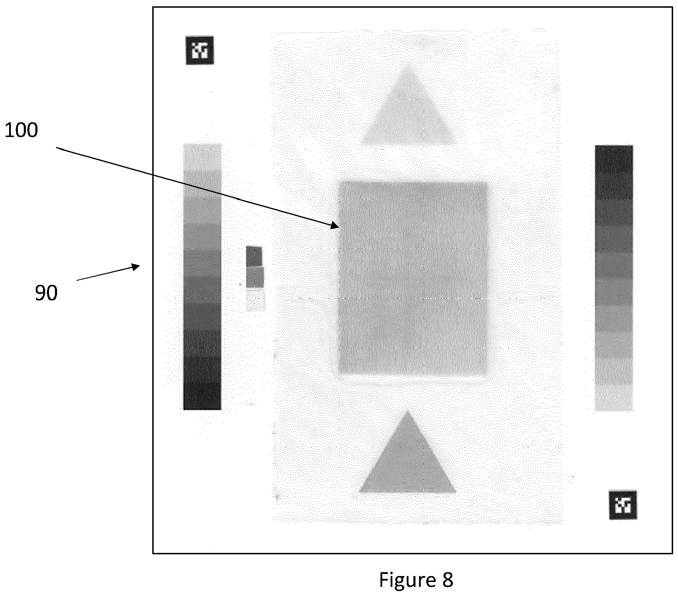

**Figure 8** is a processed image corresponding to Figure 6 in which the test patch has turned completely pink after applying to a foot for the predefined time indicating a normal level of hydrosis;

**Figure 9** is a flowchart showing the steps performed in a test at the highest level;

**Figure 10** is a flowchart showing the sequence of prompts issued by the App on the smartphone that a user must follow to perform a reliable test;

**Figure 11** is a flowchart showing the main pre-processing steps performed by the processor on a captured image of a scorecard and test patch;

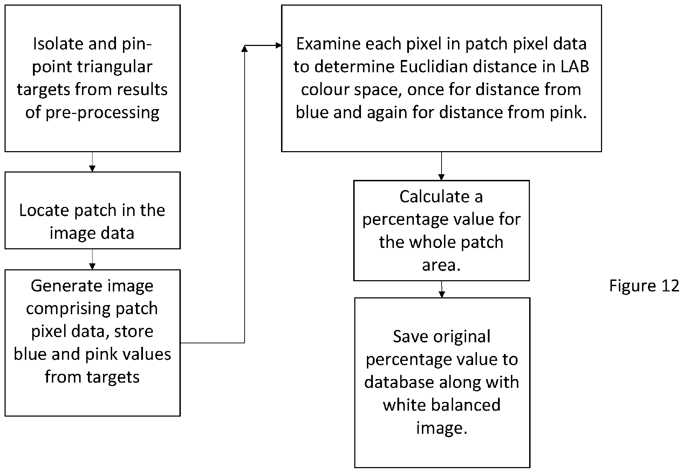

**Figure 12** is a flowchart showing how a percentage value is generated from the preprocessed images; and

**Figure 13** shows the results of application of a patch to (a) a normal foot, (b) a foot where there is some evidence of small fibre denervation, and (c) to a foot where sudomotor dysfunction has been detected.

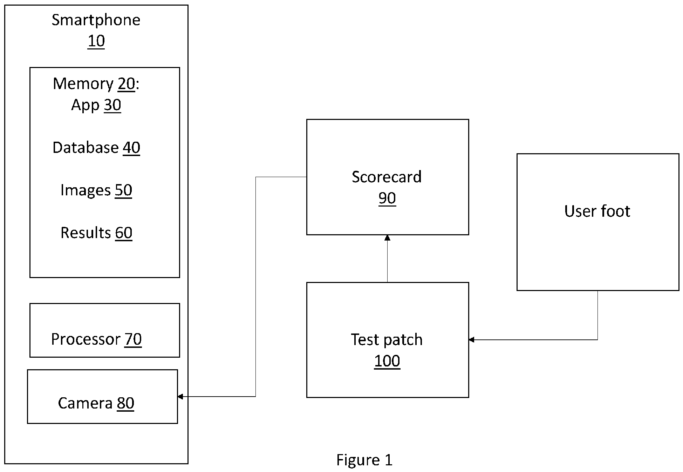

As shown in Figure 1, a system in accordance with a first aspect of the invention has three main component parts: an app 30 which runs on a smartphone 10 or another computer device, a scorecard 90 and at least one test patch 100. The smartphone 10 may be any suitable device owned by or accessible to a user, and as shown includes a processor, an area of memory 20 in which the app 30 is stored, a processor 70 and a camera 80. The skilled person will appreciate that a smartphone has other features including a display and a user interface. The later is often integrated into the display using a touch sensitive screen. The user interface enables the user to operate the device and to follow prompts generated by the App when it is running on the device.

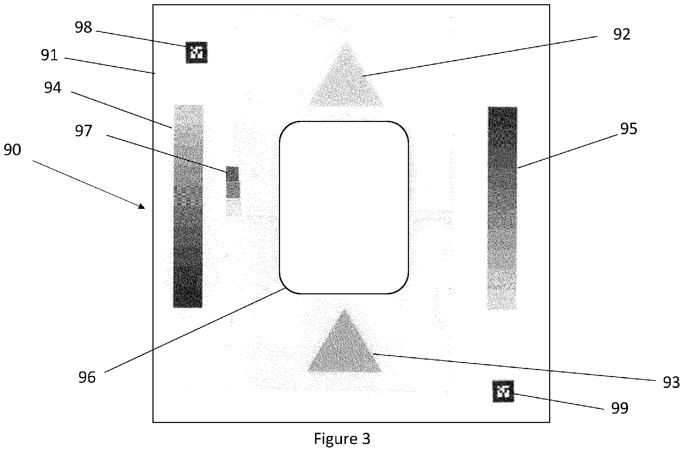

The scorecard 90 is shown in Figure 3 of the drawings and comprises a white card backing part 91. Printed on the front of the scorecard 90 are several colour reference targets. These include a first target 92 and a second target 93 that are in the shape of a triangle, although other shapes could be used The shape is intentionally selected to be unique on the scorecard to enable the location of these targets to be identified easily in the captured image. The colour of the first target 92 is blue to match the colour of a test patch that is fully dehydrated. The colour of the second target 93 is pink to match the colour of a test patch that is fully hydrated. Both have a triangular shape. The backing part 91 also carries two greyscale strips 94,95.The two targets and two greyscale strips are located around a central area which is marked with a boundary box that has the same size and shape as a test patch. This box shows where a test patch is to be fixed to the scorecard 90 by a user when carrying out a test.

The scorecard also carries a colour swatch 97 having cyan, magenta and yellow regions. Again as shown this has a unique shape allowing it to be easily identified in the image. Finally, the scorecard carries two fiducial markers 98,99 of known pattern, size and location.

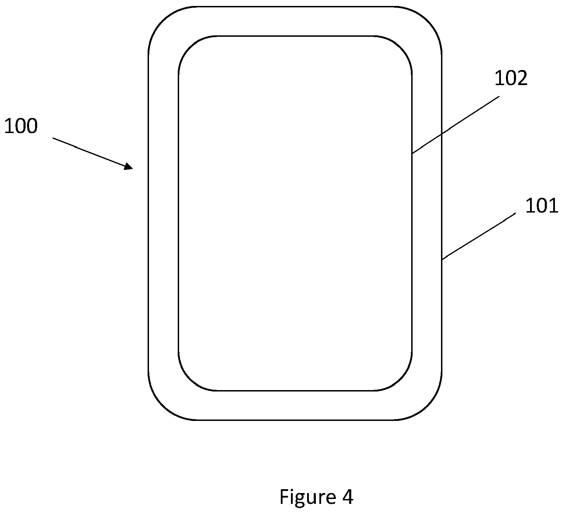

The test patch 100 comprises a flexible patch like a conventional adhesive plaster that would typically be used to protect wounds on the skin. An example patch is shown in Figure 4 of the drawings. The test patch has a planar base 101 which has a layer of adhesive (not shown) on a rear face. This enables the patch to be stuck to the skin of a user, typically onto the foot when used to detect abnormal levels of moisture on the foot. The test patch 100 has a central permeable carrier 102 impregnated with a dried cobalt(II) chloride solution. In this state the carrier has a blue colour. Prior to use the test patch 100 may be stored in a moisture proof pouch or covered by a moisture proof removable skin. When fully hydrated, as would occur over a defined period of time when stuck to a foot of a person with a normal level of hydrosis, the hydrated cobalt(II) chloride turns pink. The time taken to turn pink all over when stuck to a foot of a person with normal levels of hydrosis is determined through tests of the test patches and this time is used as the dwell time for which the patch is to remain on the foot before removal. In the case of a commercially available neuropad® test patch this time is set as 10 minutes.

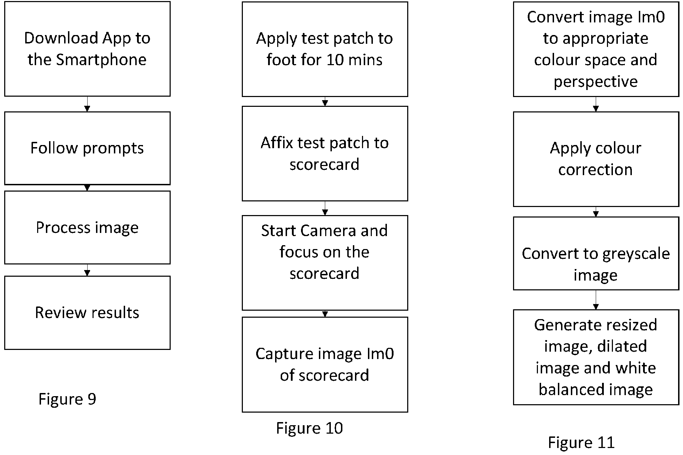

The system may be used to perform the following method steps as part of a process for determining if a user has an abnormal level of hydrosis of their foot, as would be the case if there was nerve damage due to diabetes. The steps are shown in Figure 9 and Figure 10. The start point is for the user to download the App to their smartphone if they have not already done so. They must then start the App running where it will prompt the user to follow a set of steps that ensure an accurate test is performed. Once the steps have been followed the results of the test are displayed by the smartphone.

The first prompt from the App is for the user to take a fresh test patch and apply to the foot for the predetermined duration, for example 10 minutes, before removing.

The second prompt is for the user to fix the patch to the marked area of the scorecard. The adhesive on the back of the patch will be sufficient to stick the patch to the scorecard.

The third prompt is for the user to turn on their camera and point the smartphone at the scorecard. The app will detect the edge of the scorecard and prompt the user when the scene of the scorecard is correct, i.e., not too far away or too close to the camera.

Once the scorecard is correctly imaged by the camera the App prompts the user to capture an image of the scorecard. The App then saves this to a database stored in the memory of the smartphone along with the date and time of the captured image.

Once the image is captured, the user is prompted to wait whilst the image is processed. The steps performed during processing may be as follows. The core numerical processing is done with an open-source library called OpenCV, a tried and tested piece of software with an incredibly high reputation amongst the numerical science community the world over.

Let us refer to the initial image as Im0. This image, Im0, undergoes a 'pre-processing' stage then the resulting image is passed to a further process which attempts to isolate the bounds of the patch from the captured image prior to the main processing stage.

Pre-Processing Stage

This stage converts the colour space of the uploaded image (Im0) into a colour space known as COLOR_BGR2RGB. This is because the internal software uses an OpenCV function called ' imread()' to load Im0 into memory and the ordering of the colour channels is Blue, Green, Red, but for subsequent processing we require that the colours be ordered in the more standard Red, Green, Blue. Once this is done, the image is then resized, maintaining the aspect ratio, to a height of seven hundred and fifty pixels.

A process of colour-correction is then done by calculating the white balance of the image. This colour processing uses the colour of the colour reference targets on the scorecard as seen in the captured image.

An optional process of image correction to remove the effects of perspective and focusing of the capture device may be performed by analysing the regions of the image that correspond to the fiducial markers. In this stage the image is distorted until the shape of the markers matches the known shape of the markers. For example, where the markers are square they will not be square in an image of the scorecard captured off axis but will be square in the distorted image after this processing step. They will also be brought into focus and in doing so the rest of the image of the scorecard can also be deemed to have been brought into focus.

Using the value determined from the previous step, the image is then converted to a gray scaled image and passed through an adaptive Gaussian threshold filter, then a morphological process is applied to the resulting grayscaled image to help identify the required target parts of the image. A process of dilation and erosion are used to enhance important parts of the image.

The result of the pre-processing stage is a set of three images, the initial resized copy, the initially dilated image, and the white-balanced image. These are used further down the algorithmic chain to produce the final percentage score of the source image.

Main Processing stage - Test patch Detection

The next step is to use the first two images returned from the pre-processing stage to isolate and pin-point the location of the fixed Pantone reference triangles 92,92 and of the other optional fiducial markers 98,99. These are of a known colour content and the algorithm knows to look for these patches to both correct any current image values and to also fully locate the test patch area within the image data.

The returned values from this stage are the image data containing the test patch pixel data, the blue and pink, the grayscaled and original images.

Main Processing stage- Percentage Calculation

The final step is to now calculate the percentage of the processed image that is deemed to be pink, blue, or some value in between. This is done by examining every single point in the final image frame, and then, using the previously derived values for the pink and blue triangles from the framing card, the Euclidean distance in LAB colour-space is calculated, once for the distance between the reference blue colour and the pixel under examination, and again for the reference pink colour and the pixel under examination.

The final percentage value is calculated as:

$$Percentage = \frac{pixels\ deemed\ blue}{pixels\ deemed\ blue + pixels\ deemed\ pink} * 100$$

This percentage value is stored in the database alongside the captured image for future reference. For example, this will allow a user to track the percentage over time and to see if their symptoms are becoming worse or improving.

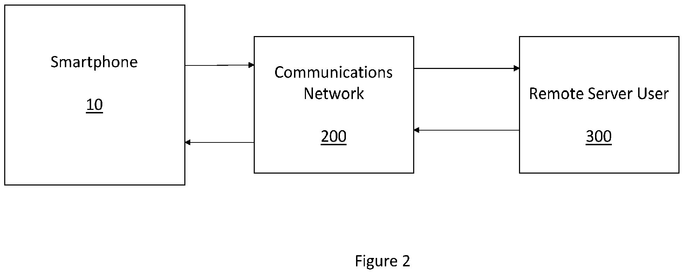

Figure 2 shows an alternative in which the smartphone 10 is connected to a remote server 300. The connection is made over a communication network 200, for instance a cellular network or a wireless or wired connection to the internet. The remote server receives captured images from the phone or the percentage results values or both. If only captured images are sent these may be stored at the server for later access by the smartphone user or a physician.

The skilled person will appreciate that many modifications are possible whilst remaining within the spirit and scope of the invention. For example, instead of a smartphone the user may capture images using any colour camera device and upload the images to a computer or tablet and transmit those images to a remote server for processing. Alternatively, a tablet device or any computer with an integral camera can be used.

**Claims**

1.  A system for use in determining a skin dryness by processing images of a test patch of the kind comprising a membrane impregnated with a moisture indicator which has a first colour in the dry anhydrous state, and which changes to a second colour at a certain moisture content which corresponds to normal skin moisture, the system comprising:

    A user operable computer device having a camera, a processor, and an area of memory, a computer program stored in the memory of the user operable device,
    a scorecard having at least one colour reference target;
    in which the computer program causes the user operable computer device to process an image of the scorecard with a test patch placed on it after the test patch has been applied to an area of skin, the processing determining the dryness of the area of the skin.

2.  A system according to claim 1 in which the moisture indicator comprises cobalt (II) chloride which has blue colour when in the dry anhydrous state and a pink colour when at the certain moisture content.

3.  A system according to claim 1 in which the colour reference target comprises a first target which has a colour that corresponds to a fully dehydrated test patch and a second target having a colour that corresponds to a fully hydrated test patch and a region in which a test patch can be placed.

4.  A system according any preceding claim in which the computer program causes the computer device to process the captured image to extract a two-dimensional image of the test patch which is colour balanced using the colours of the colour reference target or targets as seen in the captured image.
    Additionally or

5.  A system according to any preceding claim in which the colour reference target includes a colour swatch including a region of cyan colour, a region of magenta colour, and a region of yellow colour.
    In this case the system may process the regions of the image corresponding to the colour swatch so as to colour balance the image of the scorecard.

6.  A system according to any preceding claim in which the scorecard further includes one or more fiducial markers that have a known shape or pattern, optionally a known size and optionally a known location on the scorecard and the processing may use the fiducial markers to produce a modified image of the scorecard which is corrected for perspective and brought into focus.

7.  A system according to any preceding claim configured to store in a database in the memory captured images captured over a period of time using the camera, or the processed images, and to store alongside each image the results of the processing of the captured image.

8.  A system according to any preceding claim in which the computer program is configured to cause the processor to process the results in the database to determine if the abnormality is changing over time and in the even that a change is detected over time to present a message or alarm to the user.

9.  A system according to any preceding claim in which the scorecard comprises a backing portion having a front face which is provided with markings defining an area of the front face to which a test patch can be adhered.

10. A system for use in determining a skin dryness by processing images of a test patch of the kind comprising a membrane impregnated with a moisture indicator, which has a first colour in the dry anhydrous state, and which changes to a second colour at a certain moisture content which corresponds to normal skin moisture, the system comprising:

> a user operable computer device having a camera, a processor and an area of memory, a computer program stored in the memory of the user operable device,
> a scorecard having at least one colour reference target, for example a first target which has a colour that corresponds to a fully dehydrated test patch and a second target having a colour that corresponds to a fully hydrated test patch and a region in which a test patch can be placed, and ;
> in which the computer program causes the user operable computer device to capture an image of the scorecard with a test patch placed on it after the test patch has been applied to an area of skin and to transmit the captured image across a network to a remote server that is configured to process the image to determine a dryness of the area of the skin.

11. A method of determining a skin dryness by processing images of a test patch of the kind comprising a membrane impregnated with a moisture indicator, which has a first colour in the dry anhydrous state, and which changes to a second colour at a certain moisture content which corresponds to normal skin moisture, the method comprising:

> capturing an image of a test patch that has previously been stuck to an area of skin together with an image of a scorecard, the scorecard having at least one colour reference target and a region in which a test patch can be placed;
> processing the captured image to identify the colour reference target and the patch;
> calibrating the colours in the captured image using the colours of the captured target, and
> analysing the image of the patch in the captured image to determine the percentage of the patch that has the first colour and the second colour.

12. A method according to claim 11 comprising capturing the image on a portable device such as a smart phone.

13. A computer program which when operable on a user device, such as a mobile phone, causes the device to perform the method of the claim 12 or claim 13.

14. A scorecard for use in combination with a test patch of the kind comprising a membrane impregnated with a moisture indicator, which has a first colour in the dry anhydrous state, and which changes to a second colour at a certain moisture content which corresponds to normal skin moisture, , the scorecard comprising:

> a backing portion;
> at least one colour reference target, and
> and in which the backing portion includes a region that is sized to receive a test patch.

15. A scorecard according to claim 14 in which the colour reference target comprises a first reference target having a known shape and having a colour corresponding to a colour of the test patch when fully dehydrated;
a second reference target having a known shape and having a colour corresponding to a colour of the test patch when fully dehydrated,

16. A scorecard according to claim 14 or claim 15, in which the colour reference target includes a colour swatch including a region of cyan colour, a region of magenta colour, and a region of yellow colour and the method may comprise processing the regions of the image corresponding to the colour swatch so as to colour balance the image of the scorecard.

17. A scorecard according to any one of claims 14 to 16 which further includes one or more fiducial markers that have a known shape or pattern on the scorecard and the processing may use the fiducial markers to produce a modified image of the scorecard which is corrected for perspective and brought into focus.

18. A scorecard according to any one of claims 14 to 17 which includes an adhesive portion that is spaced from the two reference targets that is configured to receive and adhere to a patch.

19. A test kit comprising at least one scorecard as defined in any one of claims 14 to 18 and at least one compatible test patch.

Smartphone
10

Memory 20:
App 30

Database 40

Images 50

Results 60

Processor 70

Camera 80

Scorecard
90

User foot

Test patch
100

Figure 1

Figure 2

Figure 3

100

102

101

Figure 4

Figure 5

Figure 6

100

90

Figure 7

Figure 8

Download App to
the Smartphone

↓

Follow prompts

↓

Process image

↓

Review results

Figure 9

---

Apply test patch to
foot for 10 mins

↓

Affix test patch to
scorecard

↓

Start Camera and
focus on the
scorecard

↓

Capture image Im0
of scorecard

Figure 10

---

Convert image Im0
to appropriate
colour space and
perspective

↓

Apply colour
correction

↓

Convert to greyscale
image

↓

Generate resized
image, dilated
image and white
balanced image

Figure 11

Isolate and pin-point triangular targets from results of pre-processing

Locate patch in the image data

Generate image comprising patch pixel data, store blue and pink values from targets

Examine each pixel in patch pixel data to determine Euclidian distance in LAB colour space, once for distance from blue and again for distance from pink.

Calculate a percentage value for the whole patch area.

Save original percentage value to database along with white balanced image.

Figure 12

(a)                                    (b)                                    (c)

Figure 13

Europäisches Patentamt

European Patent Office

Office européen des brevets

# EUROPEAN SEARCH REPORT

**Application Number**

EP 23 21 0769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/166268 A1 (YAMAGUCHI HIROSHI [JP] ET AL) 10 July 2008 (2008-07-10) * paragraphs [0040] - [0057], [0066] - [0068], [0079]; figures 1,5a * | 1-19 | INV. A61B5/103 A61B5/00 |
| Y | US 2003/091472 A1 (CREMERIUS ALOIS [DE] ET AL) 15 May 2003 (2003-05-15) * paragraph [0017]; claim 1 * | 1-13,19 | |
| X | US 2021/158574 A1 (SUWA YUJI [JP] ET AL) 27 May 2021 (2021-05-27) | 14-18 | |
| Y | * paragraphs [0034] - [0041], [0057], [0072] - [0088], [0097], [0120] * | 1-13,19 | |
| X | US 2018/196037 A1 (POLWART NEIL [GB] ET AL) 12 July 2018 (2018-07-12) | 14-18 | |
| Y | * paragraphs [0047], [0112] - [0120], [0136] - [0137]; figure 10 * | 1-13,19 | |
| X | US 2020/126226 A1 (ADIRI YONATAN [IL] ET AL) 23 April 2020 (2020-04-23) | 14-18 | TECHNICAL FIELDS SEARCHED (IPC) |
| Y | * paragraphs [0073] - [0091], [0102], [0116] - [0117], [0185] * | 1-13,19 | G01J G01N A61B |

The present search report has been drawn up for all claims

| Place of search | Date of completion of the search | Examiner |
|---|---|---|
| Berlin | 14 March 2024 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 0769

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2024

| Patent document cited in search report | | | Publication date | Patent family member(s) | | | Publication date |
|---|---|---|---|---|---|---|---|
| US 2008166268 | A1 | | 10-07-2008 | JP | 2008167853 | A | 24-07-2008 |
| | | | | US | 2008166268 | A1 | 10-07-2008 |
| US 2003091472 | A1 | | 15-05-2003 | AT | 6435 | U1 | 27-10-2003 |
| | | | | AT | E344927 | T1 | 15-11-2006 |
| | | | | AU | 779351 | B2 | 20-01-2005 |
| | | | | CN | 1422383 | A | 04-06-2003 |
| | | | | DE | 10018790 | A1 | 25-10-2001 |
| | | | | DK | 1274994 | T3 | 19-03-2007 |
| | | | | EP | 1274994 | A1 | 15-01-2003 |
| | | | | ES | 2276794 | T3 | 01-07-2007 |
| | | | | PT | 1274994 | E | 31-01-2007 |
| | | | | US | 2003091472 | A1 | 15-05-2003 |
| | | | | US | 2007203409 | A1 | 30-08-2007 |
| | | | | WO | 0179836 | A1 | 25-10-2001 |
| US 2021158574 | A1 | | 27-05-2021 | CN | 112469978 | A | 09-03-2021 |
| | | | | JP | 7132041 | B2 | 06-09-2022 |
| | | | | JP | 2020038073 | A | 12-03-2020 |
| | | | | US | 2021158574 | A1 | 27-05-2021 |
| | | | | WO | 2020049779 | A1 | 12-03-2020 |
| US 2018196037 | A1 | | 12-07-2018 | BR | 112013025223 | A2 | 20-10-2020 |
| | | | | CA | 2857424 | A1 | 04-10-2012 |
| | | | | CN | 103649731 | A | 19-03-2014 |
| | | | | DK | 2646809 | T3 | 10-12-2018 |
| | | | | EP | 2646809 | A1 | 09-10-2013 |
| | | | | EP | 3470825 | A1 | 17-04-2019 |
| | | | | ES | 2696600 | T3 | 17-01-2019 |
| | | | | JP | 6189286 | B2 | 30-08-2017 |
| | | | | JP | 6480988 | B2 | 13-03-2019 |
| | | | | JP | 2014514547 | A | 19-06-2014 |
| | | | | JP | 2017215337 | A | 07-12-2017 |
| | | | | KR | 20140074256 | A | 17-06-2014 |
| | | | | KR | 20190026943 | A | 13-03-2019 |
| | | | | PT | 2646809 | T | 27-11-2018 |
| | | | | RU | 2013148400 | A | 10-05-2015 |
| | | | | US | 2014154789 | A1 | 05-06-2014 |
| | | | | US | 2018196037 | A1 | 12-07-2018 |
| | | | | WO | 2012131386 | A1 | 04-10-2012 |
| US 2020126226 | A1 | | 23-04-2020 | US | 2020126226 | A1 | 23-04-2020 |
| | | | | US | 2021241456 | A1 | 05-08-2021 |

EPO FORM P0459

For more details about this annex : see Official Journal of the European Patent Office, No. 12/82

## REFERENCES CITED IN THE DESCRIPTION

**Patent documents cited in the description**

• EP 127499 B